# FASCICULE DE BREVET EUROPEEN

(11) **EP 1 140 124 B1**
(45) Date de publication et mention de la délivrance du brevet: **11.05.2005**
(21) Numéro de dépôt: 99961160.1
(22) Date de dépôt: 23.12.1999
(51) Int. Cl.: A61K 35/78, A61P 19/00, A61P 25/00

(54) **UTILISATION D'INSAPONIFIABLES D'HUILES VEGETALES POUR LA PREPARATION D'UN MEDICAMENT**
VERWENDUNG VON UNVERSEIFBAREN PFLANZLICHEN ÖLEN FÜR DIE HERSTELLUNG PHARMAZEUTISCHER PRÄPARATE
USE OF UNSAPONIFIABLE MATTERS OF VEGETABLE OILS FOR PREPARING A MEDICINE

(30) Priorité: 23.12.1998 FR 9816328
(43) Date de publication de la demande: 10.10.2001
(73) Titulaire: Laboratoires Expanscience, 92419 Courbevoie Cedex (FR)
(72) Inventeur: BOUMEDIENE, Karim, F-14420 Ussy (FR); PUJOL, Jean-Pierre, F-14440 Douvres-la-Délivrande (FR); GUILLOU, Georges, Bernard, F-44240 La Chapelle-sur Erdre (FR); MSIKA, Philippe, F-75018 Paris (FR); GHAYOR, Chafik, Uni de Caen, Lab. de Biochimie du, F-14000 Caen (FR)
(74) Mandataire: Warcoin, Jacques
(86) Numéro de dépôt international: PCT/FR1999/003272
(87) Numéro de publication internationale: WO 2000/038701

(56) Documents cités:
- EP-A- 0 643 960
- WO-A-99/59523
- WO-A-99/63031
- FR-A- 2 231 394
- FR-A- 2 678 632
- FR-A- 2 762 512
- HELEN KIM ET AL.: "MECHANISMS OF ACTION OF THE SOY ISOFLAVONE GENISTEIN: EMERGING ROLE FOR ITS EFFECTS VIA TRANSFORMING GROWTH FACTOR BETA SIGNALING PATHWAYS." THE AMERICAN JOURNAL OF CLINICAL NUTRITION, vol. 68, no. 6(S), décembre 1998 (1998-12), pages 1418S-1425S, XP002113859
- H. THIERS: "UN GROUPE D'AGENTS THERRAPEUTIQUES NOUVEAUX: LES INSAPONIFIABLES DES HUILES VEGETALES, LEURS CAROTENOIDES, LEURS PHYTOSTEROLS ET LEURS INDETERMINES ADMINISTRES EN SOLUTION ALCOOLIQUE." THERAPIE, vol. 16, 1961, pages 235-251, XP002113860

## Description

La présente invention concerne l'utilisation d'au moins un insaponifiable d'huile végétale, notamment d'huiles d'avocat, de soja et/ou de lupin, ayant un effet stimulateur de l'expression du TGF-β pour la préparation d'un médicament destiné au traitement de pathologies sélectionnées dans le groupe composé d'arthrite de lyme, d'arthrite psoriasique et d'ostéoporose ainsi qu'une utilisation cosmétique selon laquelle on applique sur la peau, les muqueuses voisines et/ou les phanères une composition à base d'insaponifiables d'huiles végétales.

La demande de brevet FR 2 231 394 divulgue que l'insaponifiable de soja peut être utilisé en tant qu'antilipémique.

La demande de brevet EP 643 960 décrit l'utilisation de la fraction insaponifiable de l'huile de soja pour le traitement des peaux mixtes pour son activité émolliente et inhibitrice de lipase.

L'article de H. Thiers et al., « un groupe d'agents thérapeutiques nouveaux : les insaponifiables des huiles végétales, leurs caroténoïdes, leurs phytostérols et leurs indéterminés administrés en solution alcoolique », Thérapie, vol. 16, 1961, pages 235-251 décrit notamment l'utilisation de l'insaponifiable de palme sous forme de pommade, pour son action eutrophique, de l'insaponifiable d'avocat pour lutter contre les lésions dyskératosiques ou encore pour lutter contre les radiodermites.

La demande de brevet FR 2 762 512 décrit des compositions à base d'huile de lupin comprenant une ou plusieurs factions d'huiles de lupin sous forme d'insaponifiable et leur utilisation cosmétique ou pharmaceutique, notamment pour la photoprotection, contre le vieillissement (actinique ou non) et pour la protection de la peau contre les agressions oxydantes.

La demande de brevet FR 2 678 632 a trait à un procédé de préparation d'un insaponifiable d'huile d'avocat, des médicaments le contenant, ainsi que des compositions cosmétiques ou additifs alimentaires, pouvant être utiles pour l'entretien du bon fonctionnement des cartilages osseux, et pour la prévention du vieillissement cutané, de l'arthrose ou des parodontopathies.

Enfin la demande internationale WO 99 59523, qui n'est opposable qu'au titre de la nouveauté, décrit des compositions pharmaceutiques topiques contenant en tant que principe actif un mélange d'extraits de pollen et d'insaponifiables d'huile végétale.

Par " TGF-β" on entend selon l'invention les différentes isoformes du TGF-β c'est-à-dire les isoformes du facteur β de croissance transformant ("Transforming Growth Factor-β"). Les isoformes du TGF-β constituent une famille de polypeptides homodimériques d'environ 25 kD de poids moléculaire. Parmi les 5 isoformes connues, les mieux caractérisées sont les TGF-β1 et TGF-β2 (Sporn et al (1987), J. Cell Biol. 105, 1039-1045 ; Roberts et Sporn (1990), Handbook of Exp. Pharmacol, 35, 419-472, Springer Verlag, Heidelberg). Bien que ces deux isoformes ne montrent que 71% d'homologie, elles semblent avoir beaucoup d'activités communes. Le TGF-β1 a d'abord été isolé à partir des plaquettes humaines, mais on sait désormais que la plupart des cellules sont capables de l'exprimer. Le TGF-β2 a été purifié à partir des plaquettes, de l'os bovin et de cellules de glioblastome.

On sait que le TGF-β est impliqué dans des mécanismes complexes d'évolution de diverses pathologies et qu'il est souhaitable de renforcer l'action du TGF-β, en d'autres termes d'augmenter son expression par les cellules elles-mêmes impliquées dans les mécanismes desdites pathologies, pour une évolution favorable de ces dernières.

Ainsi, par exemple, il est connu que le TGF-β exprime par les chondrocytes articulaires est impliqué dans des mécanismes de réaction anaboliques, c'est-à-dire de restauration, du cartilage articulaire observés aux premiers stades de l'arthrose et qui tendent à compenser la dégradation du cartilage résultant de l'activité de métalloprotéases secrétées de manière excessive par les chondrocytes sous l'effet des cytokines, telles que que l'interleukine-1 (IL-1).

Par ailleurs, il est connu que le TGF-β est favorablement impliqué dans les mécanismes de remodelage osseux qui interviennent au cours de l'ostéoporose. Ceci a été montré en particulier par Boyce et al de l'Université du Texas ((1996), Nature Med. (2), 10, 1132-1136.).

Enfin, le TGF-β joue également un rôle favorable dans certains mécanismes de différenciation de cellules nerveuses induits par le facteur de croissance des nerfs (NGF ou "Nerve Growth Factor") ainsi que dans de nombreux aspects de la réparation tissulaire, en particulier cutanée.

Par ailleurs, par "inhibiteur PAI-1 de l'activateur du plasminogène" on entend selon l'invention l'inhibiteur spécifique PAI-1 qui, avec l'autre inhibiteur PAI-2, régule de manière connue l'activité de la forme tissulaire (tPA) et du type urokinase (uPA) de l'activateur du plasminogène PA. Les deux formes de PA, le tPA et l'uPA, sont produites par deux gènes différents et ont des poids moléculaires ainsi qu'une réactivité immunologique différents (Dano et al, (1985) Adv. Cancer Res. 44, 139-166 ; Hart et al, (1988), Comp. Bioch. Physiol. 90 B, 691-708). Les inhibiteurs PAI-1 et PAI-2 forment des complexes stables avec le tPA et l'uPA. PAI-1 est la forme majoritaire dans le plasma et est produit par les cellules endothéliales, les plaquettes et les cellules de l'articulation telles que les cellules synoviales et les chondrocytes (Hart et al, 1988 ; Campbell et al, 1991 ; Hamilton et al, 1992).

Il serait particulièrement avantageux de pouvoir stimuler l'expression de l'inhibiteur PAI-1 de l'activateur du plasminogène, dans la mesure où l'on obtiendrait ainsi une inhibition de l'action des métalloprotéases et donc, en particulier, une contribution à l'action du TGF-β pour une évolution favorable des pathologies citée ci-aprés.

On a maintenant constaté de manière tout à fait surprenante et inattendue que l'utilisation d'insaponifiables d'huile végétable permet d'obtenir non seulement un effet de stimulation de l'expression du TGF-β mais aussi un effet de stimulation de l'expression de l'inhibiteur PAI-1 de l'activateur du plasminogène.

Ainsi, la présente invention concerne l'utilisation d'au moins un insaponifiable d'huile végétale ayant un effet stimulateur de l'expression du TGF-β pour la préparation d'un médicament destiné au traitement de pathologies sélectionnées dans le groupe composé d'artrite de lyme, d'arthrite psoriasique et d'ostéoporose.

En particulier, l'utilisation selon l'invention est caractérisée par le fait que le médicament est destiné à stimuler l'expression du TGF-β, et plus particulièrement l'expression des isoformes TGF-β1 et TGF-β2.

Plus particulièrement, comme cela ressort clairement de l'exemple 1 ci-après, l'utilisation selon l'invention est caractérisée par le fait que le médicament est destiné à stimuler l'expression du TGF-β par l'intermédiaire des séquences d'ADN situées entre -1132 et -732 paires de bases (pb) du promoteur du TGF-β et notamment du promoteur de l'isoforme TGF-β1.

L'utilisation selon l'invention est également caractérisée par le fait que le médicament est destiné à stimuler l'expression de l'inhibiteur PAI-1 de l'activateur du plasminogène.

D'une manière générale, l'insaponifiable est la fraction d'un corps gras qui, après action prolongée d'une base alcaline, reste insoluble dans l'eau et peut être extraite par un solvant organique. Cinq grands groupes de substances sont présents dans la plupart des insaponifiables d'huiles végétales : hydrocarbures saturés ou insaturés, alcools aliphatiques ou terpéniques, stérols (ou "phytostérols"), tocophérols et tocotriénols, les pigments caroténoïdes et xanthophiles.

De préférence, l'insaponifiable d'huile végétale utilisée selon l'invention est choisi dans le groupe constitué par l'insaponifiable d'huile d'avocat, l'insaponifiable d'huile de soja, l'insaponifiable d'huile de lupin et les mélanges de ces derniers.

La comparaison des teneurs en insaponifiables de différentes huiles végétales : soja, coton, noix de coco, olive et avocat montre un taux très important d'insaponifiable de l'huile d'avocat obtenue par extraction suivant divers procédés connus. Typiquement, les teneurs obtenues s'échelonnent de 2 à 7% d'insaponifiable dans l'huile d'avocat contre 0,5% dans l'huile de coco, 1% dans l'huile de soja, 1% dans l'huile d'olive.

La teneur plus importante en insaponifiable dans l'huile d'avocat par rapport aux autres huiles végétales telles que celles mentionnées ci-dessus s'explique en particulier par la présence, dans l'insaponifiable d'huile d'avocat, de constituants que l'on ne retrouve généralement pas dans l'insaponifiable de nombreuses autres huiles végétales tels que des composés furaniques et des alcools gras polyhydroxylés et qui, à eux seuls, représentent plus de 50% de l'insaponifiable. Les produits propres à cet insaponifiable d'avocat peuvent être répartis en deux fractions chimiques appelées "fraction I" et "fraction H". Les composés actifs pour l'utilisation selon l'invention se trouvent notamment présents dans la fraction H et ses précurseurs. La fraction H apparaît en premier lieu sur un chromatographe en phase gazeuse de l'insaponifiable d'huile d'avocat.

L'insaponifiable d'huile d'avocat utilisé selon l'invention peut être obtenu à partir du fruit frais mais, de préférence, l'insaponifiable d'huile d'avocat utilisé selon l'invention est l'insaponifiable d'huile d'avocat séché (c'est-à-dire l'insaponifiable obtenu à partir de l'huile du fruit avocat séché).

Selon l'invention, l'insaponifiable d'avocat comprend de préférence au moins sa fraction enrichie en dérivés furaniques (fraction H), sa fraction enrichie en alcools gras polyhydroxylés (fraction I) ou un mélange de ces fractions.

Concernant l'insaponifiable d'huile de soja, on peut remarquer que cet insaponifiable est principalement composé de stérols (40 à 65%) et de tocophérols (≥ 10%). Les principaux stérols sont le β-sitostérol (40 à 70% des stérols totaux), le campéstérol (15 à 30% des stérols totaux) et le stigmastérol (10 à 25% des stérols totaux). Les tocophérols sont présents sous la forme d'un mélange de α-tocophérol (5 à 35% des tocophérols totaux), de γ-tocophérol (45 à 70% des tocophérols totaux) et de δ-tocophérol (10 à 43% des tocophérols totaux).

L'huile de lupin peut être extraite à partir de farines et/ou de graines de lupin.

Le lupin est un proche parent du pois, de la fève, du soja et du haricot. La graine est traditionnellement employée en alimentation humaine pour sa forte teneur en protéines. Il est également incorporé dans l'alimentation des ruminants sous forme de la plante entière ou de ses graines et aussi fréquemment utilisé comme engrais vert. Plus particulièrement, quatre espèces de lupin présentent un réel intérêt agronomique : le lupin blanc (lupinus albus), le lupin bleu (lupinus angustifolius), le lupin jaune (lupinus luteus) et le lupin changeant (lupinus mutabilis).

On a constaté que l'huile de lupin possède une teneur particulièrement élevée en dérivés polyphénoliques, carotènes (notamment β-carotène) et tocophérols.

Selon l'invention, on préfère également utiliser tout insaponifiable d'huile végétale contenant des fractions riches en phytostérols, tocophérols, tocotriénols, hydrocabures terpéniques et tripterpéniques, des antioxydants naturels, en particulier l'insaponifiable d'huile de canola, de colza, de tournesol, de palme, de maïs, de sésame, de germe de blé, l'insaponifiable d'huile de soja et les mélanges de ces derniers. L'homme du métier comprend aisément que le terme "riche" fait référence à des teneurs en ces différents composants cités respectivement au-dessus des teneurs moyennes respectives obtenues en considérant l'ensemble des huiles végétales connues de l'homme du métier.

Plusieurs procédés ont été décrits dans l'art antérieur pour extraire la fraction insaponifiable d'une huile végétale.

On peut citer en particulier le procédé de préparation d'insaponifiable d'huile d'avocat tel que décrit et revendiqué dans le brevet FR-2 678 632 au nom des Laboratoires Pharmascience. Ce procédé permet d'obtenir un insaponifiable d'avocat riche en fraction H en comparaison aux procédés classiques de préparation d'insaponifiable d'avocat.

On peut également citer le procédé de préparation d'insaponifiable d'huile de soja, obtenu à partir d'un concentrat d'insaponifiable d'huile de soja. Ledit concentrat d'insaponifiable est préparé par distillation moléculaire selon un procédé tel que décrit pour l'huile de lupin dans la demande de brevet FR-2 762 512, mais adapté à l'huile de soja. Dans ce procédé, l'huile de soja est distillée dans un distillateur moléculaire de type centrifuge ou à film raclé, à une température comprise entre environ 210 et 250° C et sous un vide poussé, compris entre 0.01 et 0.001 millimètres de mercure (soit 0,13 à 1,3 Pa). Le distillat obtenu présente une teneur en insaponifiable comprise entre 5 et 30% en poids et constitue donc un concentrat d'insaponifiable d'huile de soja. Le même concentrat est ensuite saponifié selon un procédé classique de saponification, en présence de potasse éthanolique. Le mélange obtenu est extrait par le dichloroéthane dans une colonne à contre-courant. La phase solvant est enfin désolvantée par passage dans un évaporateur à film tombant afin de récupérer l'insaponifiable de soja.

Comme exemple de procédé de préparation d'insaponifiable d'huile de lupin, on peut citer celui décrit dans la demande de brevet FR- 2 762 512. On se référera en particulier à l'exemple 3 de cette demande.

Selon un mode de réalisation préféré de la présente invention, l'insaponifiable d'huile végétale est un mélange d'insaponifiables d'huiles d'avocat et de soja, le rapport pondéral d'insaponifiable d'huile d'avocat à l'insaponifiable d'huile de soja étant compris entre environ 0,1 et environ 9, et de préférence compris entre environ 0,25 et environ 0,6.

En particulier, on peut avantageusement utiliser le mélange d'insaponifiables d'huiles d'avocat et de soja tel que commercialisé par la société Laboratoires Pharmascience sous la dénomination "Piascledine 300®" qui consiste en un mélange de 33,3% en poids d'insaponifiable d'avocat et de 66,6% en poids d'insaponifiable de soja, par rapport au poids total du mélange (les 0,1% restants étant constitués de silice colloïdale et de butylhydroxytoluène).

De préférence, on utilise l'insaponifiable d'huile végétale selon l'invention de telle sorte qu'il soit présent dans le médicament selon une proportion comprise entre environ 1 et environ 80% en poids, par rapport au poids total du médicament.

Le médicament préparé par l'utilisation selon la présente invention peut ainsi comprendre en outre un excipient pharmaceutiquement acceptable, de préférence adapté à une administration par voie orale, topique externe, entérale ou parentérale.

Plus particulièrement, ce médicament comprend un excipient adapté à une administration par voie orale.

Le médicament préparé par l'utilisation selon l'invention, du fait de son action stimulant l'expression du TGF-β et de son action stimulant l'expression de l'inhibiteur PAI-1 de l'activateur du plasminogène, est donc avantageusement destiné aux traitements des pathologies citées ci-dessus.

La présente invention concerne enfin également une utilisation, autre qu'une méthode de traitement thérapeutique, d'au moins un insaponifiable d'huile végétale choisi dans les groupe constitué par l'insaponifiable d'huile d'avocat, l'insaponifiable d'huile de soja, l'insaponifiable d'huile de lupin et les mélanges de ces derniers pour la préparation d'une composition cosmétique comprenant au moins un véhicule cosmétiquement acceptable, à appliquer sur la peau, les muqueuses voisines et/ou les phanères et destinée au traitement cosmétiquement des cicatrices de la peau, des muqueuses voisines et/ou des phanères, à la condition que la composition cosmétique ne comprenne pas d'extrait de pollen.

En outre, la présente invention concerne une utilisation, autre qu'une méthode de traitement thérapeutique, d'au moins un insaponifiable d'huile végétale choisi dans le groupe constitué par l'insaponifiable d'avocat, l'insaponifiable d'huile de soja et les mélanges de ces derniers pour la préparation d'une composition cosmétique comprenant au moins un véhicule cosmétiquement acceptable, à appliquer sur la peau, les muqueuses voisines et/ou les phanères et destinée au traitement du vieillissement intrinsèque de la peau, des muqueuses voisines et/ou des phanères, à la condition que la composition cosmétique ne comprenne pas d'extrait de pollen.

Enfin, elle concerne l'utilisation, autre qu'une méthode de traitement thérapeutique, d'au moins un insaponifiable d'huile végétale choisi dans le groupe constitué par l'insaponifiable d'huile d'avocat, l'insaponifiable d'huile de soja et les mélanges de ces derniers pour la préparation d'une composition cosmétique comprenant au moins un véhicule cosmétiquement acceptable, à appliquer sur la peau, les muqueuses voisines et/ou les phanères et destinée au traitement cosmétique de la peau, des muqueuses voisines et/ou des phanères ayant été soumis à un rayonnement actinique, à la condition que la composition cosmétique ne comprenne pas d'extrait de pollen.

Par ailleurs, l'exemple 2 ci-après illustre une utilisation selon l'invention à savoir pour stimuler la biosynthèse du collagène, notamment par des fibroblastes dermiques. Plus particulièrement, l'utilisation selon l'invention est caractérisée par le fait que le médicament est destiné à la reconstruction de la matrice extracellulaire, et est plus particulièrement encore destiné au traitement des troubles de la matrice extracellulaire liés au vieillissement cutané.

Par ailleurs, il est connu que le TGF-β agit au niveau du cycle pilaire en modulant, dans le sens d'une inhibition, la repousse du poil. En outre, selon un mécanisme encore mal élucidé, il s'avère que le TGF-β exerce une action sur les tissus folliculaires dont l'effet est de provoquer la chute du poil. Ces deux actions présentent donc un intérêt cosmétique évident dans le domaine de l'épilation. En particulier, les effets dépilatoires complémentaires (inhiber la repousse du poil et provoquer la chute du poil) permettent avantageusement à l'utilisateur d'espacer dans le temps les séances d'épilation classiques plus contraignantes et souvent plus coûteuses, y compris les séances de rasage mécanique notamment chez l'homme. Cette utilisation cosmétique peut par exemple s'envisager sous forme d'un baume après-épilation ou après-rasage conjuguant les effets cosmétiques classiques de ce type de baume (hydratation, effet apaisant, etc.) et ces effets dépilatoires.

Ainsi, la présente invention a encore pour objet l'utilisation, autre qu'une méthode de traitement thérapeutique, d'au moins un insaponifiable d'huile végétale choisi dans le groupe constitué par l'insaponifiable d'huile d'avocat, l'insaponifiable d'huile de soja, l'insaponifiable d'huile de lupin et les mélanges de ces derniers pour la préparation d'une composition cosmétique destinée au traitement cosmétique dépilatoire de la peau, à appliquer sur la peau, et comprenant au moins un véhicule cosmétiquement acceptable.

De préférence, l'insaponifiable d'huile végétale est présent dans la composition cosmétique selon une proportion comprise entre environ 0,1 et environ 10 % en poids, par rapport au poids total de la composition cosmétique.

La présente invention va maintenant être illustrée à l'aide d'exemples qui ne doivent en aucun cas être interprétés comme pouvant en limiter la portée.

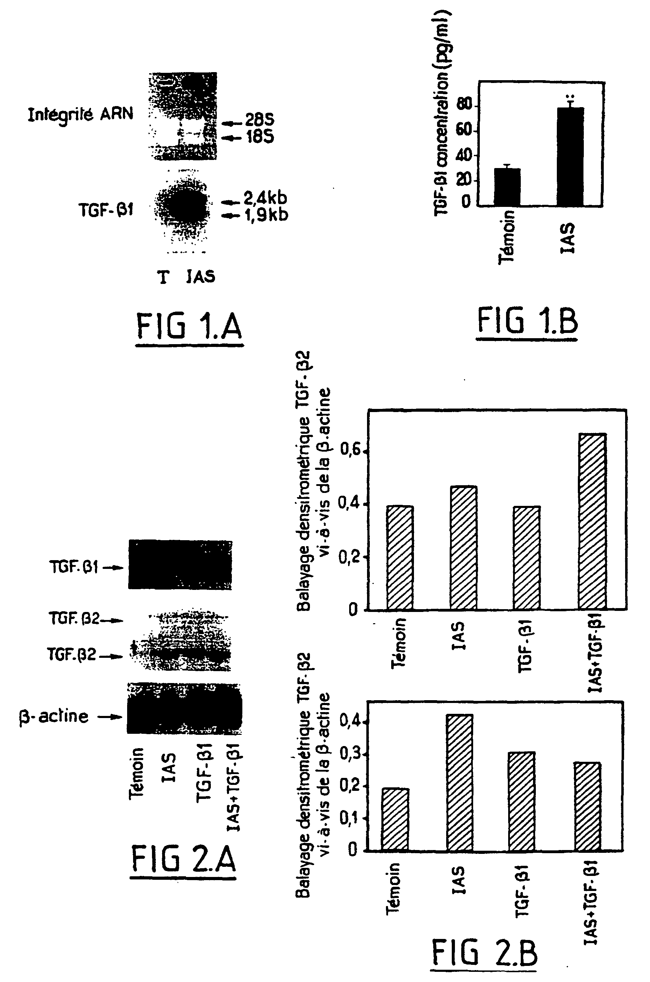

La **figure 1** (c'est à dire les **figures 1.A et 1.B**) représente un cliché d'une expérience de northern-blot **(figure 1.A)** montrant l'expression accrue de l'ARNm de TGF-β1 dans les cellules traitées aux insaponifiables d'avocat et de soja (Piasclédine 300® ; référencé "IAS" dans les figures 1 à 6) ainsi qu'un histogramme **(figure 1.B)** correspondant à un dosage protéique montrant l'expression protéique accrue de TGF-β1 dans les cellules traitées avec Piasclédine 300® (le témoin est référencé "T" dans les figures 1 à 6).
La **figure 2** (c'est à dire les **figures 2.A et 2.B**) montre les effets des insaponifiables d'avocat et de soja seuls, du TGF-β1 seul et de la combinaison [insaponifiables d'avocat et de soja et TGF-β1] sur l'expression des ARNm des isoformes TGF-β1 et TGF-β2 comme expliqué à la fin de l'exemple 1 ci-après, paragraphe 2.1. En particulier, les clichés de northern-blot **(figure 2.A)** montrent l'expression de l'ARNm de TGF-β1, TGF-β2 et de la β-actine prise comme témoin. Les résultats de l'expression des ARNm respectivement des isoformes TGF-β1 et TGF-β2 sont représentés sous forme des histogrammes respectifs **(figure 2.B)**.

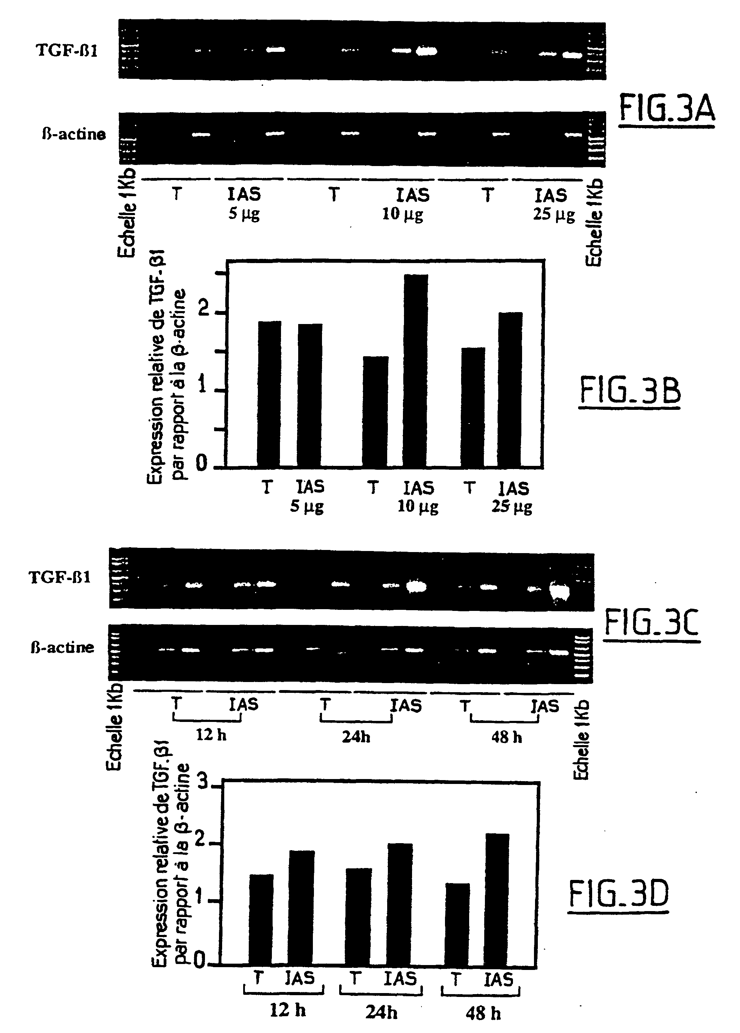

La **figure 3** (c'est à dire les **figures 3.A, 3.B, 3.C, et 3.D**) illustre les effets au cours du temps et selon les doses utilisées des insaponifiables d'avocat et de soja (Piasclédine 300®) sur l'expression de TGF-β1. La **figure 3.A** est une photographie d'un gel d'électrophorèse de produit d'amplification RT-PCR à partir d'ARNm de TGF-β1 de cellules traitées par différentes quantités d'insaponifiables d'avocat et de soja (Piasclédine 300®). Les résultats sont normalisés et exprimés sous forme d'histogramme **(figure 3.B)**. La **figure 3.C** est une photographie d'un gel d'électrophorèse de produit d'amplification RT-PCR à partir d'ARNm de TGF-β1 de cellules traitées à des temps différents par les insaponifiables d'avocat et de soja (Piasclédine 300®). Les résultats sont normalisés et exprimés sous forme d'histogramme **(figure 3.D)**.

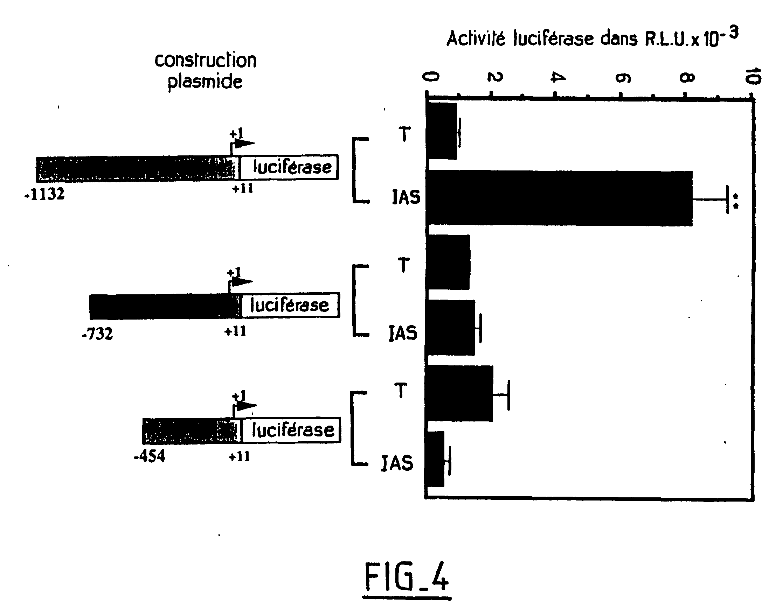

La **figure 4** montre les effets des insaponifiables d'avocat et de soja (Piasclédine 300®) sur l'expression de la luciférase en fonction de différentes constructions de promoteur de TGF-β1.

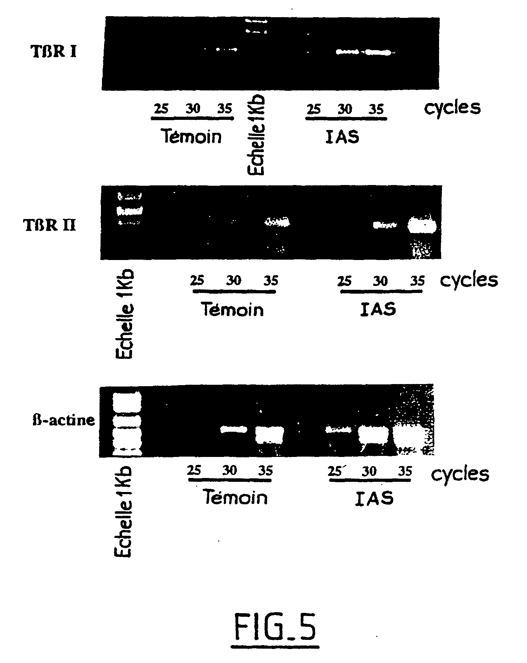

La **figure 5** est une photographie d'un gel d'électrophorèse de produit d'amplification RT-PCR à partir d'ARNm des récepteurs TGF-β1 et TGF-β2 et à partir d'ARNm de β-actine prise comme témoin, provenant de cellules traitées par les insaponifiables d'avocat et de soja (Piasclédine 300®).

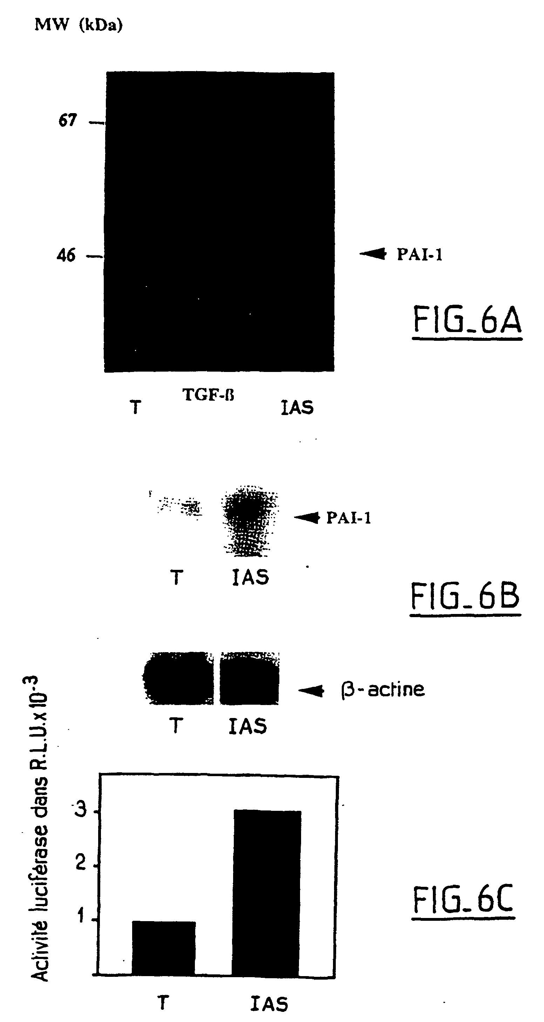

La **figure 6** (c'est à dire les **figures 6.A, 6.B et 6.C**) illustre l'effet des insaponifiables d'avocat et de soja (Piasclédine 300®) sur l'expression de l'inhibiteur PAI-1 de l'activateur du plasminogène. La **figure 6.A** représente une photographie d'un gel d'électrophorèse de protéine obtenu par extraction à partir de cellules traitées par Piasclédine 300® ou par TGF-β. La **figure 6.B** est une photographie de northern-blot montrant l'expression d'ARN de PAI-1 dans les cellules traitées par Piasclédine 300®. Les résultats de l'expérience de northern-blot sont normalisés et exprimés sous forme de l'histogramme à la **figure 6.C.**

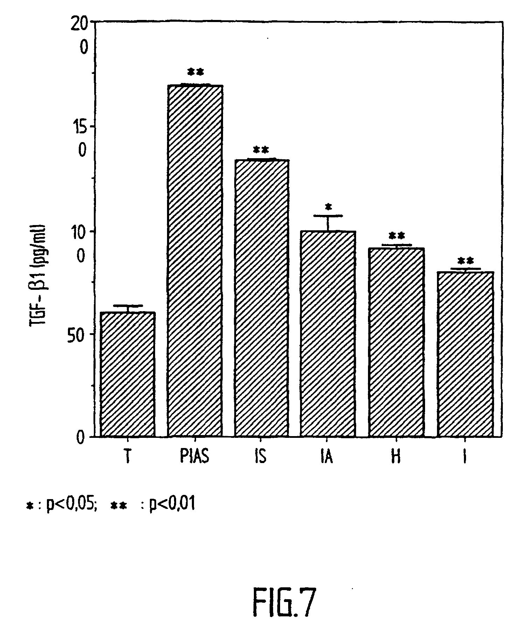

La **figure 7** montre l'effet des insaponifiables d'avocat et de soja et des fractions H et I sur l'expression du TGF-β1.

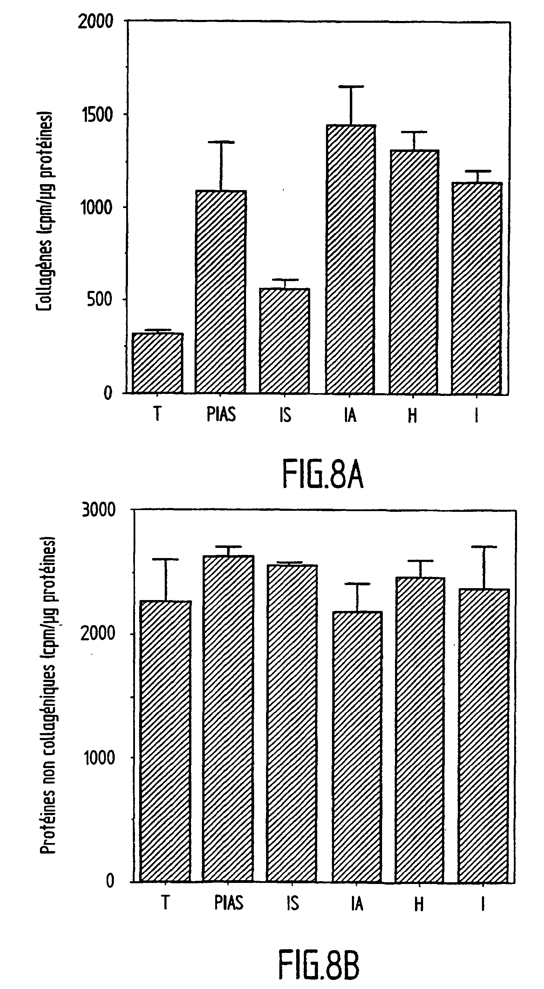

La **figure 8** (c'est à dire les **figures 8A et 8B**) montre l'effet des insaponifiables d'avocat et de soja et des fractions H et I sur la biosynthèse de collagène **(figure 8A)** et de protéines non collagéniques **(figure 8B)**, par des fibroblastes dermiques en culture.

### Exemple 1 : Effet des insaponifiables d'avocat et de soja sur l'expression du TGF-β1 et TGF-β2 et sur l'expression du PAI-1

### 1.1. Matériels et Méthodes

### 1.1.1. Culture et traitement de chondrocytes articulaires

Les chondrocytes sont isolés à partir de cartilages de veaux comme décrit dans l'article de Benya et al ("The progeny of articular chondrocytes synthesize collagen types I et II trimer, but not type II. Verification by cyanogen bromide peptide analysis", Biochemistry 1977 ; 16 : 865-872). Des cultures primaires de chondrocytes sont utilisées afin de minimiser les changements phénotypiques. Les cultures sont étalées à raison de 6,0 x 10⁶ cellules par boîtes de 175 cm² (pour l'extraction d'ARN) à raison de 5,0 x 10⁵ cellules par puits dans des plaques à 6 puits (9,6 cm²) (pour le marquage du PAI-1) et à raison de 1,2 x 10⁶ cellules dans des boîtes de Pétri de 100 mm (transfection). Les cultures sont incubées dans du milieu complet DMEM (DMEM pour "Dulbecco's modified Eagle's medium") contenant 10% de sérum de veau foetal (FCS) et des antibiotiques à 35° C dans une atmosphère à 5% de CO₂ et 95% d'air, jusqu'à atteindre la confluence (à l'exception des essais de transfection). Les cultures sont incubées en présence d'insaponifiables d'avocat et de soja sous la forme du produit "Piasclédine 300®" (commercialisé par la société Laboratoires Pharmascience) qui contient 33,3% d'insaponifiable d'huile d'avocat et 66,6% d'insaponifiable d'huile de soja. La concentration de Piasclédine 300® étant de 10 µg/ml, et en présence de TGF-β1 à raison de 1 ng/ml (commercialisé par la société R & D Systems) pendant les temps indiqués. Dans la mesure où Piasclédine 300® est dissout dans du diméthylformamide (DMF), on inclut dans tous les essais des témoins contenant la même concentration en DMF.

### 1.1.2. Extraction d'ARN

L'ARN total est extrait par la méthode de solubilisation différentielle au phénol/chloroforme en utilisant le kit commercial RNAXel de la société Eurobio. Les concentrations en ARN sont déterminées par la mesure de la valeur de la densité optique à 260 nm (DO₂₆₀). Les rapports DO₂₆₀/DO₂₈₀ sont supérieurs à 1,8. L'intégrité des échantillons d'ARN est contrôlée par électrophorèse sur gel d'agarose à 1% en présence de bromure d'éthidium. Dans le cas d'une contamination d'ADN génomique, on effectue une précipitation supplémentaire avec du chlorure de lithium LiCl 6M pour obtenir des échantillons purs d'ARN.

### 1.1.3. Hybridation de l'ARN (Northern-blot)

On fait passer 10 µg d'échantillons d'ARN dénaturé sur un gel d'agarose formaldéhyde à 1%. L'ARN est transféré par capillarité sur une membrane nylon (Pall Biodyne, Gelman Sciences) et immobilisé par irradiation aux ultra-violets (Bioblock UV Crosslinker, France). On utilise trois sondes différentes pour évaluer les valeurs d'ARNm pour le TGF-β, le PAI-1, le β-actine : (a) on génère une sonde d'ADNc à 336 paires de bases (pb) pour le TGF-β1 par RT-PCR (reverse transcription et réaction en chaîne à la polymérase) en utilisant les amorces indiquées ci-dessous, (b) un fragment d'ADNc de 3000 pb correspondant au PAI-1 humain (fourni par le laboratoire du Dr J-P Pelletier, Montréal, Canada) et (c) une sonde de 548 pb de β-actine générée par RT-PCR avec les amorces spécifiques énumérées ci-dessous. Les sondes d'ADNc sont radiomarquées en utilisant le kit de marquage aléatoire des amorces (Gibco BRL, France) et [³²P]-dCTP en tant que radiomarqueur (Amersham, France). Chaque sonde est hybridée séparément à 55° C et lavée deux fois pendant 20 minutes à température ambiante et une fois à 55° C avec un tampon 2 x SSC contenant du SDS à 0,1%. On détecte les signaux par autoradiographie à contact de film en utilisant un film Kodak (X-OMAT AR5) avec des écrans intensifiants. La densité optique relative des signaux autoradiographiques est normalisée vis-à-vis des valeurs de la β-actine en utilisant la technique de densitométrie par balayage laser à deux dimensions et le logiciel ImageQuaNt (Molecular Dynamics, France).

### 1.1.4. Analyse RT-PCR

Des échantillons de 1 µg d'ARN total sont reverse transcrits en ADNc en présence d'une amorce antisens 100pM, 10 unités de "RNasin®" (un inhibiteur de ribonucléase commercialisé par la société Promega), 10 mM de dithiotréitol, 0,5 mM de chaque triphosphate désoxynucléotide (dNTPs) (Life Technologies), un premier tampon "Strand" 5X et 60 unités de reverse transcriptase du virus Moloney de la leucémie murine (Life Technologies). La réaction est réalisée à 42° C pendant une heure. L'amplification de l'ADNc généré est réalisée dans un thermocycleur Omni E Hybaid en utilisant le kit PCR de Life Technologies, en présence des amorces sens et antisens : TGF-β1, sens 5'- GCC CTG GAC ACC AAC TAT TGC-3'/antisens 5'- GCT GCA CTT GCA GGA GGG CAC-3' (Lupparello et al, "Transforming Growth Factor-β1, -β2 and -β3, urokinase and parathyroid hormone-related peptide expression in 8701-Bc breast cancer cell and clones, Differenciation, 1993; 55: 73-80) ; TβR-1, sens 5'-ATT GCT GGA CCA GTG TGC TTC GTC-3'/antisens 5'- TAA GTC TGC AAT ACA GCA AGT TCC ATT CTT-3' (Franzen et al), "Cloning of TGF-β type I receptor that forms a heteromeric complex with the TGF-β type II receptor" Cell, 1992; 75: 681-692); TβR-II, sens 5'- CGC TTT GCT GAG GTC TAT AAG GCC-3'/antisens 5'- GAT ATT GGA GCT CTT GAG GTC CCT-3' (Lin Hy et al, "Expression cloning of the TGF-β type II receptor, a functional transmembrane serine/threonine kinase" Cell, 1992; 68: 775-785); β-actine, sens 5'- GTG GGG CGC CCC AGG CAC CA-3'/antisens 5'- CTC CTT AAT GTC ACG CAC GAT TTC-3' (Lupparello et al référencé ci-dessus). On a effectué 35 cycles en utilisant les conditions suivantes : 95° C pendant 30 secondes, 55° C pendant 30 secondes et 72° C pendant 1 minute. Ensuite, on inclue une étape supplémentaire à 72° C pendant 10 minutes. Le nombre de cycles est choisi dans la phase exponentielle de la courbe d'amplification établie précédemment. Les transcrits ont été analysés par électrophorèse sur gel d'agarose à 2% et visualisés par coloration au bromure d'éthidium. Les réactions d'amplification ont fourni des tailles attendues de transcrit TGF-β1 : 336 pb, TβR-I: 668 pb, TβR-II: 454 pb, β-actine: 548 pb). L'identité des produits de la PCR est également confirmée par digestion par endonucléase de restriction et par Southern-blot, en utilisant les sondes humaines respectives qui correspondent, pour le TβR-I et TβR-II, à la longueur totale des ADNc et pour TGF-β1, β-actine aux fragments générés par RT-PCR. Après photographie des gels avec un film polaroïd 665, l'intensité des bandes correspondantes est quantifiée par balayage densitométrique réalisé avec le logiciel ImageQuaNt (Molecular Dynamics) et normalisée vis-à-vis des valeurs d'ARNm β-actine.

### 1.1.5. Mesure du TGF-β1 mature sécrété

Afin de mesurer la quantité de TGF-β1 actif dans le milieu conditionné par les cellules témoins contrôle ou les cellules traitées aux insaponifiables d'avocat et de soja, les cellules monocouches confluantes dans les plaques de culture à 6 puits sont incubées pendant 24 heures dans le milieu contenant du FCS à 10%, avec ou sans 10 µg de Piasclédine 300®. Les cellules sont lavées à trois reprises avec un milieu exempt de sérum supplémenté avec 200 µg/ml de BSA (sérum-albumine bovine) pendant 5, 30 et 60 minutes, et incubées dans 1 ml du milieu exempt de sérum pendant 6 heures supplémentaires. Le milieu conditionné est ensuite recueilli dans des tubes siliconés pour microcentrifugation, centrifugés et la quantité de TGF-β1 mature, activé, est déterminée dans le surnageant par immunodosage du TGF-β1 en utilisant le kit Quantikine® (Quantikine® R&D Systems, U.S.A.) selon les instructions du fabricant.

### 1.1.6. Radiomarquage pour la synthèse de PAI-1

Les cultures confluentes dans des plaques à 6 puits (9,6 cm²) sont marquées à la méthionine [³⁵S] (40 µCi/ml, Amersham, France) en présence de Piasclédine 300® (10 µg/ml) et de TGF-β1 (1 ng/ml) pendant 24 heures. L'extraction des protéines radiomarquées et la caractérisation du PAI-1 par électrophorèse sont réalisées comme décrit dans l'article de Laiho M et al " Transforming Growth Factor-β induction of type-1 plasminogen activator inhibitor" J. Biol. Chem. 1987; 262: 17467-17474) avec quelques modifications. Brièvement, les milieux sont retirés et les couches de cellules sont raclées dans 1 ml de tampon à pH 8 Tris-HCl à 10 mM, contenant 0,5% de déoxycholate de sodium et 1 mM de fluorure de phénylméthane-sulfonyle. Les échantillons sont centrifugés à 4° C. 10 000g pendant 10 minutes. Les surnageants sont absorbés avec de la concanavaline A (Con A)-Sépharose (Pharmacia) (50 µl de 50% (v/v) de suspension dans du PBS). La Con A-Sépharose est lavée à trois reprises avec un mélange PBS/Tween 80 (0,01%) et les protéines fixées sont dissoutes dans du tampon de Laemmli (Laemmli et al, "Cleavage of structural proteins during the assembly of the head of bacteriophage T₄" Nature, 1970 ; 227: 680-685) contenant 10% de 2-mercaptoéthanol. Les protéines fixées sur la concanavaline A sont soumises à une électrophorèse sur gel de polyacrylamide 10% en présence de dodécyl-sulfate de sodium polyacrylamide (SDS-PAGE) suivi d'une fluorographie. L'ovalbumine (Mᵣ 46 000) et l'anhydrase carbonique (Mᵣ 29 000) sont utilisées comme marqueur de poids moléculaire. La bande à 46 kD est déterminée comme correspondant au PAI-1 par immunoblotting précédent en utilisant un anticorps anti-PAI-1 polyclonal de lapin (Dako, Copenhague, Danemark) tel que décrit dans l'article de Laiho M et al référencé ci-dessus.

### 1.1.7. Transfection cellulaire et essai d'activité à la luciférase

Pour les transfections transitoires, on étale les cellules dans des boîtes de Petri de 100 mm et on les fait croître jusqu'à 70-80% de confluence. Les cellules sont ensuite cotransfectées par la méthode de coprécipitation au phosphate de calcium (Bradford et al, "A rapid and sensitive method for the quantification of microgram quantities of protein utilizing the principle of protein-dye binding", Anal. Biochem, 1976 ; 72: 248-254), avec 9 µg de plasmides appropriés et 3 µg de pSV40-β Gal (Promega), un vecteur d'expression de la β-galactosidase utilisé en tant que témoin interne pour normaliser l'efficacité de la transfection. Après 24 heures, le milieu est remplacé par un milieu contenant du DMF (1:1000) en l'absence ou en présence de Piasclédine 300® (10 µg/ml).

Les cellules sont récoltées 48 heures après l'addition d'ADN et les extraits sont testés quant à l'activité à la luciférase. Brièvement, les boîtes de Petri sont lavées à deux reprises avec du PBS, et les cellules sont lysées avec 300 µl de tampon de lyse (0,45 mM Tris-HCl, pH 7,5). Les lysats sont soumis à trois cycles de congélation-décongélation. Après centrifugation, on mesure la luminescence d'un aliquot de 50 µl de lysat de chaque boîte de Petri dans un luminomètre (Berthold Lumat 9501) pendant 20 secondes après addition de luciférine (Luciferase Assay System, société Proméga). Afin de normaliser l'activité de la luciférase, on détermine la concentration de protéine et l'activité de la β-galactosidase. La concentration de protéine de lysats cellulaires de 4µl de chaque boîte de Petri est mesurée selon Bradford tel que référencé ci-dessus. Les lysats cellulaires sont testés quant à l'activité de la β-galactosidase en utilisant la résofurine β-D galactopiranoside en tant que substrats et on mesure la DO à 572 nm.

### 1.1.8. Construction des plasmides

Les constructions contenant le promoteur du TGF-β1 sont générées à partir du plasmide phTG2 (Kim et al, "Characterization of the promotor region of the human transforming growth factor-β1 gene", J. Biol. Chem. 1989; 264: 402-408) fourni par le docteur S.J. Kim (Laboratoire de Chemoprevention, NIH/NCI; Bethesda). La digestion du plasmide phTG2 avec les enzymes HindIII et XlaI respectivement génère des fragments d'ADN correspondant aux séquences promotrices respectives -1132 à +11 et -732 à +11. Après remplissage des extrémités cohésives par le fragment de Klenow, les fragments d'ADN sont digérés par KpnI et sont ensuite clonés dans le plasmide reporter standard pGL2 au site SmaI/KpnI (Promega) codant pour le gène de la luciférase sans séquence promotrice. La construction contenant la région promotrice -454/+11 correspond au clonage d'une séquence d'ADN obtenue par digestion de phTG2 par HindII- et KpnI dans un site SmaI-KpnI du plasmide reporter standard pGL2.

### 2. Résultats

### 2.1. Effet des insaponifiables d'avocat et de soja (Piasclédine 300®) sur l'expression du TGF-β1 et TGF-β2

L'effet de Piasclédine 300® sur les chondrocytes articulaires bovins est déterminé dans des cultures primaires confluentes incubées pendant 24 heures en présence ou en absence de Piasclédine 300® (10 µg/ml), les témoins contenant la même concentration de diméthylformamide (DMF 1:1000) qui est utilisé comme solvant de l'extrait. Aucun changement morphologique ou détachement cellulaire n'a été obversé avec les concentrations jusqu'à 100 µg/ml, comme examiné par microscopie à contraste de phase. Toutefois, la concentration de 10 µg/ml a été selectionnée pour la plupart des essais dans la mesure où la valeur de DMF dans les échantillons à 100 µg/ml aurait été trop élevée et potentiellement nuisible pour les cellules. L'expression des TGF-β1 et TGF-β2 a été déterminée après extraction de l'ARN total par northern-blot.

Comme le montre la figure 1, le traitement de BAC (Bovine Articular Chondrocytes) avec Piasclédine 300® à 10 µg/ml a provoqué une augmentation notable du niveau d'ARNm TGF-β1 par rapport aux témoins, lorsque le message était à peine détectable. Deux transcrits de 1,9 et 2,4 kb ont été observés sur les northern-blots. Cette expression augmentée du gène TGF-β1 induit par Piasclédine 300® est spécifique puisque l'expression de l'ARNm de β-actine qui correspond à un gène de ménage, n'a pas changée de manière significative sous les mêmes conditions expérimentales.

Afin de déterminer si cet effet de transcription est accompagné d'une augmentation de la synthèse des protéines TGF-β, un dosage ELISA permet d'estimer la concentration de TGF-β1 libéré dans les milieux des cultures cellulaires traitées par Piasclédine 300®. La figure 1 montre que la production de TGF-β1 immunodétectable est augmentée par une exposition de 24 heures à Piasclédine 300® 10 µg/ml. Bien que l'essai ne permet pas de distinguer le TGF-β latent du TGF-β activé, il résulte clairement qu'il existe une corrélation entre l'effet des insaponifiables d'avocat et de soja sur la transcription et la traduction et sur l'expression du TGF-β1.

Dans une seconde série d'expérience, on a examiné les effets de Piasclédine 300® sur la valeur de l'expression tant du TGF-β1 que TGF-β2 en présence ou en l'absence de TGF-β1 exogène. Comme le montre la figure 2, l'expression du TGF-β2 a été également stimulée par Piasclédine 300®. De manière intéressante, dans le cas du TGF-β1, on a observé un effet de synergie montrant que des boucles d'amplification peuvent survenir dans le système.

### 2.2. Effet (temps- et doses- dépendances) des insaponifiables d'avocat et de soja sur l'expression du TGF-β1 en fonction du temps et des doses

Puisque ces résultats montrent que Piasclédine 300® induit une augmentation de l'expression du TGF-β, on a voulu déterminer les effets de concentrations différentes des insaponifiables d'avocat et de soja et des temps d'incubation différents sur le taux d'expression des ARNm de TGF-β1. Dans la mesure où le signal détecté par la méthode northern-blot est relativement faible pour les chondrocytes témoins non traités (voir la figure 1), on a préféré utiliser la méthode RT-PCR pour réaliser ces essais. Le traitement de chondrocytes au Piasclédine 300® à des concentrations croissantes (5, 10 et 25 µg/ml) permet d'observer une réponse à la stimulation pour des concentrations en Piasclédine 300® de 10 et 25 µg/ml avec un effet plus important pour une concentration de 10 µg/ml. La concentration de 5 µg/ml est probablement trop faible pour produire un effet quelconque (voir la figure 3). Le traitement de chondrocytes au Piasclédine 300® à une concentration de 10 µg/ml pendant des périodes de 12, 24 ou 48 heures, permet d'observer une augmentation du taux d'expression des ARNm de TGF-β1 à toutes les périodes d'incubation avec un maximum à 48 heures (voir la figure 3).

### 2.3. Effet des insaponifiables d'avocat et de soja sur l'expression cellulaire dirigée par la région 5' flanquantes du gène TGF-β1

D'après les résultats précédents qui montrent que les insaponifiables d'avocat et de soja peuvent stimuler l'activité de transcription du promoteur du gène TGF-β1, l'objet de cet essai est de délimiter les séquences en cis du gène TGF-β1 susceptibles de médier cet effet. Une série de fragments de la région 5'- du promoteur du gène humain du TGF-β1 fusionnés au gène de la luciférase sont transfectés dans des chondrocytes bovins ensuite traités pendant 24 heures avec Piasclédine 300® à 10 µg/ml. L'expression de l'activité luciférase est ensuite testée pour chaque plasmide. Comme le montre la figure 4, la contruction la plus longue utilisée (-1132 à +11) induit une expression d'activité luciférase 8 fois supérieure à celle du témoin. Les autres séquences correspondant à la région la plus en aval (-732 à +11) ne produisent pas de changement significatif quant à l'expression de la luciférase comparée à celle des témoins.

Ces résultats montrent que les séquences d'ADN répondant à la stimulation des insaponifiables d'avocat et de soja sur l'expression du gène de TGF-β1 sont situées entre -1132 et -732.

### 2.4. Effet des insaponifiables d'avocat et de soja sur l'expression des récepteurs du TGF-β

Dans la mesure où l'activité biologique du TGF-β est médiée via sa liaison aux récepteurs membranaires cellulaires, on se propose d'étudier les effets des insaponifiables d'avocat et de soja sur l'expression des récepteurs du TGF-β en mesurant les niveaux d'ARNm correspondants. Comme le montre la figure 5, le traitement au Piasclédine 300® n'induit aucune variation du taux d'expression des ARNm de TβR-I et de TβR-II. L'analyse de ces données par balayage densitrométrique et normalisation par rapport au signal de la β-actine montre que le rapport relatif pour le TβR-I établi avec l'amplification à 35 cycles, est de 1,16 dans les cultures traitées en comparaison avec la valeur de 1,23 dans les témoins et la valeur de 0,83 pour le TβR-II vis-à-vis de 0,70 dans les témoins.

### 2.5. Effet des insaponifiables d'avocat et de soja sur l'expression du PAI-1

Dans les cultures monocouches confluentes des chondrocytes utilisés dans ces essais, des essais précédents ont montré que le PAI-1 produit par les cellules a été essentiellement trouvé dans la couche de cellule, qui comprend une matrice environnante abondante. On a constaté que le milieu contenait seulement des quantités mineures de PAI-1 néosynthétisé. Par conséquent, on a extrait le PAI-1 marqué à la méthionine- ³⁵S à partir de la fraction cellules + matrice sans distinguer entre la partie intracellulaire et la partie extracellulaire du PAI-1 total produit. Comme le montre la figure 6, lorsque l'on a traité des cultures de chondrocytes de bovin avec du TGF-β1 pendant 24 heures, la fraction de PAI-1 isolée par électrophorèse sur gel a été significativement plus importante que celle des cultures témoins. Ce résultat a fourni la preuve que les cellules répondaient au TGF-β1 en terme d'expression du PAI-1 et a validé le système utilisé pour déterminer l'effet des insaponifiables d'avocat et de soja. Dans les mêmes conditions, il est clair que les extraits d'avocat et de soja ont pu augmenter la synthèse de PAI-1, pratiquement selon un même degré que l'a fait le TGF-β1 (voir la figure 6).

Afin de déterminer si l'effet de stimulation des insaponifiables de soja et d'avocat sur la synthèse de PAI-1 s'exerce au niveau transcriptionnel on a réalisé un northern-blot à partir d'ARN total isolé à partir de cultures traitées de la même manière que pour le marquage de la protéine PAI-1. L'hybridation avec la sonde ADNc de PAI-1 marquée au phosphore 32 a démontrée que la quantité d'ARNm codant pour PAI-1 est augmentée pendant les 24 heures du traitement aux insaponifiables d'avocat et de soja, révélant la corrélation existant entre le taux d'expression de l'ARNm et le niveau de protéine (voir la figure 6). Il est ainsi suggéré que les insaponifiables d'avocat et de soja sont capables d'augmenter la synthèse de PAI-1 au niveau transcriptionnel.

### Exemple 2 : Effet des insaponifiables d'avocat et de soja et des fractions H et I sur l'expression du TGF-β1 et sur la biosynthèse de collagène par des fibroblastes dermiques en culture

Les cellules utilisées pour cette étude sont des fibroblastes humains issus de prépuce de jeunes enfants. Ils ont été obtenus par la technique d'explants et cultivés dans du DMEM ("Dulbecco's Modified Eagle Medium") additionné de 10% de sérum de veau foetal (SVF). Les cultures sont maintenues dans une étuve à 5% de CO2, 37°C et le milieu est changé tous les trois jours.

### 1. Effet des insaponifiables d'avocat et de soja et des fractions H et I sur l'expression du TGF-β1

### 1.1 Protocole expérimental

Des fibroblastes dermique humains ont été ensemencés dans des boîtes de six puits de 9,6 cm² dans du DMEM additionné de 10% de sérum de veau foetal (SVF) (3 ml). A confluence, les cultures de fibroblastes ont été pré-incubées avec du DMEM + 2% SVF pendant 24 heures. L'incubation proprement dite est réalisée dans du DMEM + 0% SVF afin d'éviter que le TGF-β présent dans le sérum n'interfère avec le dosage. Ce milieu est additionné de 1,5 ml des différentes préparations testées, à savoir un contrôle (C), Piasclédine 300^{R} (PIAS) un insaponifiable de soja (IS) un insaponifiable d'avocat (IA) et ses fractions enrichies en dérivés furaniques (H) et en alcools gras polyhydroxylés (I). Le dosage est réalisé en utilisant un kit ELISA (R&D systems).

### 1.2 Résultats

Le dosage du TGF-β1 est réalisé sur le milieu de culture en suivant les instructions du fournisseur. Une gamme étalon est réalisée en utilisant des quantités connues de TGF-β1, ce qui permet de tracer une droite de la densité optique (DO) par rapport à la concentration et de déterminer l'équation de la droite ainsi que le coefficient de corrélation. La concentration de TGF-β1 dans nos échantillons est calculée en utilisant les DO obtenues avec les différentes préparations testées. Les résultats sont exprimés en pg/ml de TGF- β1 et représentent la moyenne de 3 échantillons.

Ces résultats présentés dans la **figure 7** montrent que PIAS, IS, IA, H, et I stimulent la production du TGF-β1 par les fibroblastes dermiques en culture.

### 2. Effet des insaponifiables d'avocat et de soja et des fractions H et I sur la biosynthèse de collagène par des fibroblastes dermiques en culture

### 2.1 Protocole expérimental

Des cultures confluentes de fibroblastes dermiques (6 puits de 9,6 cm²) sont préincubées avec de l'acide ascorbique (3ml). Après 24 heures, le milieu est remplacé par 1,5 ml de milieu (DMEN/10% SVF additionné de β/APN (aminopropionitrile), d'acide ascorbique et de ³H-Proline (2 µCi/ml).

La biosynthèse de collagènes a été mesurée par la technique de dosage à la collagénase bactérienne purifiée décrite par Peterkofsky et Diegelmann (1971).

24 h après le traitement des cultures avec les différentes préparations testées, à savoir un contrôle (C), Piasclédine 300R (PIAS) un insaponifiable de soja (IS) un insaponifiable d'avocat (IA) et ses fractions enrichies en dérivés furaniques (H) et en alcools gras polyhydroxylés (I), à 20 µg/ml (solubilisées au préalable dans de l'éthanol absolu), le dosage est effectué sur le milieu de culture car, en présence d'aminoproprionitrile, la majeure partie du collagène synthétisé (95%) par les fibroblastes en culture est présent sous forme soluble.

### 2.2 Résultats.

Les résultats sont présentés dans les **figures 8A** et **8B**. Comme le montre la **figure 8A**, les préparations PIAS, IS, IA, H et I augmentent la biosynthèse du collagène par des fibroblastes dermiques humains pour des concentrations de 20 µg/ml.

Les effets observés sont spécifiques des collagènes puisqu'il n'y a pas d'effet sur la biosynthèse des protéines non collagéniques, comme le montre la **figure 8B.**

L'ensemble de ces résultats montre que les substances FIAS, IS, IA, H et I stimulent la biosynthèse de collagène par les fibroblastes dermiques en culture.

### 3. Conclusion

Les résultats de cette étude montrent que les insaponifiables d'avocat et soja (PIAS IS, IA, H et I) stimulent la biosynthèse du collagène par des fibroblastes dermiques en culture. De plus, ces effets sont spécifiques du collagène dans la mesure où il n'y a pas d'effet sur la biosynthèse des protéines non collagéniques.

Par ailleurs, les préparations utilisées augmentent la production de TGF-β1 par les fibroblastes dermiques.

Ce dernier résultat indique que la stimulation de la biosynthèse du collagène par les différentes préparations passerait par une voie impliquant le TGF-β1.

Les résultats obtenus dans cette étude montrent que les insaponifiables d'avocat et soja (PIAS, IS, IA, H et I) sont capables d'augmenter la biosynthèse du collagène, principale molécule de la matrice extracellulaire (MEC) produite par les fibroblastes dermiques. De plus, ces préparations augmentent la production du TGF-β1, puissant stimulant de la synthèse des principales macromolécules de la MEC.

Les insaponifiables d'avocat et soja, par leur action sur la biosynthèse du collagène et du TGF-β1, présentent donc un potentiel important pour la reconstruction de la MEC, notamment dans le phénomène du vieillissement cutané.

## Revendications

1. Utilisation d'au moins un insaponifiable d'huile végétale ayant un effet stimulateur de l'expression du TGF-β pour la préparation d'un médicament destiné au traitement de pathologies sélectionnées dans le groupe composé d'arthrite de lyme, d'arthrite psoriasique et d'ostéoporose.

2. Utilisation selon la revendication 1 **caractérisée par le fait que** le médicament est destiné à stimuler l'expression des isoformes TGF-β1 et TGF-β2, par l'intermédiaire des séquences d'ADN situées entre - 1132 et -732 pb du promoteur de l'isoforme TGF-β1.

3. Utilisation selon les revendications 1 ou 2 **caractérisée en ce que** ledit médicament est destiné à stimuler également l'expression de l'inhibiteur PAI-1 de l'activateur du plasminogène.

4. Utilisation selon les revendications 1 à 3, **caractérisée par le fait que** l'insaponifiable d'huile végétale est choisi dans le groupe constitué par l'insaponifiable d'huile d'avocat, l'insaponifiable d'huile de soja, l'insaponifiable d'huile de lupin et les mélanges de ces derniers.

5. Utilisation selon la revendication 4, **caractérisée par le fait que** l'insaponifiable d'huile végétale est l'insaponifiable d'huile d'avocat.

6. Utilisation selon la revendication 5, **caractérisée en ce que** l'insaponifiable d'huile végétale est l'insaponifiable d'huile d'avocat séché.

7. Utilisation selon la revendication 5 ou 6, **caractérisée par le fait que** l'insaponifiable d'avocat comprend au moins sa fraction enrichie en dérivés furaniques (fraction H), sa fraction enrichie en alcools gras polyhydroxylés (fraction I) ou un mélange de ces fractions.

8. Utilisation selon la revendication 4, **caractérisée par le fait que** l'insaponifiable d'huile végétale est l'insaponifiable d'huile de soja.

9. Utilisation selon la revendication 4, **caractérisée par le fait que** l'insaponifiable d'huile végétale est l'insaponifiable d'huile de lupin.

10. Utilisation selon la revendication 4, **caractérisée par le fait que** l'insaponifiable d'huile végétale est un mélange d'insaponifiable d'huile d'avocat et d'insaponifiable d'huile de soja, le rapport pondéral d'insaponifiable d'huile d'avocat à l'insaponifiable d'huile de soja étant compris entre environ 0,1 et environ 9.

11. Utilisation selon l'une quelconque des revendications 1 à 3, **caractérisée par le fait que** l'insaponifiable d'huile végétale contient des fractions riches en phytostérols, tocophérols, tocotriénols, hydrocabures terpéniques et triterpéniques, et des antioxydants naturels.

12. Utilisation selon la revendication 11, **caractérisée par le fait que** l'insaponifiable d'huile végétale est choisi dans le groupe constitué par l'insaponifiable d'huile de canola, de colza, de tournesol, de palme, de mais, de sésame, de germe de blé, l'insaponifiable d'huile de soja et les mélanges de ces derniers.

13. Utilisation selon l'une quelconque des revendications précédentes, **caractérisé par le fait que** l'insaponifiable d'huile végétale est présent dans le médicament selon une proportion comprise entre environ 1 et environ 80% en poids, par rapport au poids total du médicament.

14. Utilisation selon l'une quelconque des revendications précédentes, **caractérisé par le fait que** le médicament comprend en outre un excipient, pharmaceutiquement acceptable, adapté à une administration par voie orale, topique externe, entérale ou parentérale.

15. Utilisation selon l'une quelconque des revendications précédentes, **caractérisé par le fait que** le médicament comprend en outre un excipient, pharmaceutiquement acceptable, adapté à une administration par voie orale.

16. Utilisation, autre qu'une méthode de traitement thérapeutique, d'au moins un insaponifiable d'huile végétale tel que défini à la revendication 4 pour la préparation d'une composition cosmétique comprenant au moins un véhicule cosmétiquement acceptable, à appliquer sur la peau, les muqueuses voisines et/ou les phanères et destinée au traitement cosmétique des cicatrices de la peau, des muqueuses voisines et/ou des phanères, à la condition que la composition cosmétique ne comprenne pas d'extraits de pollen.

17. Utilisation, autre qu'une méthode de traitement thérapeutique, d'au moins un insaponifiable d'huile végétale choisi dans le groupe constitué par l'insaponifiable d'huile d'avocat, l'insaponifiable d'huile de soja et les mélanges de ces derniers pour la préparation d'une composition cosmétique comprenant au moins un véhicule cosmétiquement acceptable, à appliquer sur la peau, les muqueuses voisines et/ou les phanères et destinée au traitement du vieillissement intrinsèque de la peau, des muqueuses voisines et/ou des phanères, à la condition que la composition cosmétique ne comprenne pas d'extrait de pollen.

18. Utilisation, autre qu'une méthode de traitement thérapeutique, d'au moins un insaponifiable d'huile végétale choisi dans le groupe constitué par l'insaponifiable d'huile d'avocat, l'insaponifiable d'huile de soja et les mélanges de ces derniers pour la préparation d'une composition cosmétique comprenant au moins un véhicule cosmétiquement acceptable, à appliquer sur la peau, les muqueuses voisines et/ou les phanères et destinée au traitement cosmétique de la peau, des muqueuses voisines et/ou des phanères ayant été soumis à un rayonnement actinique, à la condition que la composition cosmétique ne comprenne pas d'extrait de pollen.

19. Utilisation selon l'une quelconque des revendications 16 à 18, **caractérisée par le fait que** l'insaponifiable d'huile végétale est l'insaponifiable d'huile d'avocat.

20. Utilisation selon l'une quelconque des revendications 16 à 18, **caractérisée en ce que** l'insaponifiable d'huile végétale est l'insaponifiable d'huile d'avocat séché.

21. Utilisation selon la revendication 19 ou 20, **caractérisée par le fait que** l'insaponifiable d'avocat comprend au moins sa fraction enrichie en dérivés furaniques (Fraction H), sa fraction enrichie en alcools gras polyhydroxylés (Fraction I) ou un mélange de ces fractions.

22. Utilisation selon l'une quelconque des revendications 16 à 18, **caractérisée, par le fait que** l'insaponifiable d'huile végétale est l'insaponifiable d'huile de soja.

23. Utilisation selon l'une quelconque des revendications 16 à 18, **caractérisée, par le fait que** l'insaponifiable d'huile végétale est un mélange d'insaponifiable d'huile de d'avocat et d'insaponifiable d'huile de soja, le rapport pondéral d'insaponifiable d'huile d'avocat et d'insaponifiable d'huile de soja étant compris entre 0,1 et environ 9.

24. Utilisation selon l'une quelconque des revendications 16 à 18, **caractérisée, par le fait que** l'insaponifiable d'huile végétale contient des fractions riches en phytostérols, tocophérols, tocotriénols, hydrocarbures terpéniques et tri-terpéniques et des antioxydants naturels.

25. Utilisation, autre qu'une méthode de traitement thérapeutique, d'au moins un insaponifiable d'huile végétale tel que défini à l'une quelconque des revendications 4 à 11 pour la préparation d'une composition cosmétique destinée au traitement cosmétique dépilatoire de la peau, à appliquer sur la peau, et comprenant au moins un véhicule cosmétiquement acceptable.

26. Utilisation selon l'une quelconque des revendications 16 à 25 **caractérisée par le fait que** l'insaponifiable d'huile végétale est présent dans la composition selon une proportion comprise entre environ 0,1, et environ 10% en poids, par rapport au poids total de la composition cosmétique.

## Patentansprüche

1. Verwendung von wenigstens einem unverseifbaren Anteil eines pflanzlichen Öls mit einer stimulierenden Wirkung auf die Expression von TGF-β für die Herstellung eines Arzneimittels, welches für die Behandlung von Pathologien, ausgewählt aus der Gruppe bestehend aus Lyme-Krankheit, Arthritis psoriatica und Osteoporose, bestimmt ist.

2. Verwendung nach Anspruch 1, **dadurch gekennzeichnet, dass** das Arzneimittel dazu bestimmt ist, die Expression der Isoformen TGF-β1 und TGF-β2 durch das Zwischenglied der DNA-Sequenzen, die zwischen -1132 und -732 bp des Promotors der Isoform TGF-β1 gelegen sind, zu stimulieren.

3. Verwendung nach den Ansprüchen 1 oder 2, **dadurch gekennzeichnet, dass** das Arzneimittel dazu bestimmt ist, gleichfalls die Expression des Inhibitors PAI-1 des Plasminogenaktivators zu stimulieren.

4. Verwendung nach den Ansprüchen 1 bis 3, **dadurch gekennzeichnet, dass** der unverseifbare Anteil eines pflanzlichen Öls aus der Gruppe bestehend aus dem unverseifbaren Anteil von Avocadoöl, dem unverseifbaren Anteil von Sojaöl, dem unverseifbaren Anteil von Lupinenöl und den Mischungen von diesen Letzteren ausgewählt wird.

5. Verwendung nach Anspruch 4, **dadurch gekennzeichnet, dass** der unverseifbare Anteil eines pflanzlichen Öls der unverseifbare Anteil von Avocadoöl ist.

6. Verwendung nach Anspruch 5, **dadurch gekennzeichnet, dass** der unverseifbare Anteil eines pflanzlichen Öls der unverseifbare Anteil von Öl von getrockneter Avocado ist.

7. Verwendung nach Anspruch 5 oder 6, **dadurch gekennzeichnet, dass** der unverseifbare Anteil von Avocado wenigstens dessen an furanischen Derivaten angereichterte Fraktion (Fraktion H), dessen an polyhydroxylierten Fettalkoholen angereicherte Fraktion (Fraktion I) oder eine Mischung von diesen Fraktionen umfasst.

8. Verwendung nach Anspruch 4, **dadurch gekennzeichnet, dass** der unverseifbare Anteil eines pflanzlichen Öls der unverseifbare Anteil von Sojaöl ist.

9. Verwendung nach Anspruch 4, **dadurch gekennzeichnet, dass** der unverseifbare Anteil eines pflanzlichen Öls der unverseifbare Anteil von Lupinenöl ist.

10. Verwendung nach Anspruch 4, **dadurch gekennzeichnet, dass** der unverseifbare Anteil eines pflanzlichen Öls eine Mischung des unverseifbaren Anteils von Avocadoöl und des unverseifbaren Anteils von Sojaöl ist, wobei das Gewichtsverhältnis des unverseifbaren Anteils von Avocadoöl zu dem unverseifbaren Anteil von Sojaöl zwischen ungefähr 0,1 und ungefähr 9 eingeschlossen liegt.

11. Verwendung nach einem der Ansprüche 1 bis 3, **dadurch gekennzeichnet, dass** der unverseifbare Anteil eines pflanzlichen Öls an Phytosterolen, Tocopherolen, Tocotrienolen, terpenischen und triterpenischen Kohlenwasserstoffen und natürlichen Antioxidationsmitteln reiche Fraktionen enthält.

12. Verwendung nach Anspruch 11, **dadurch gekennzeichnet, dass** der unverseifbare Anteil eines pflanzlichen Öls aus der Gruppe bestehend aus dem unverseifbaren Anteil von Canola-, Colza-, Sonnenblumen-, Palm-, Mais-, Sesam-, Weizenkeimöl, dem unverseifbaren Anteil von Sojaöl und den Mischungen von diesen Letzteren ausgewählt wird.

13. Verwendung nach einem der vorangegangenen Ansprüche, **dadurch gekennzeichnet, dass** der unverseifbare Anteil eines pflanzlichen Öls in dem Arzneimittel entsprechend einem Anteil zwischen ungefähr 1 und ungefähr 80 Gew.-% eingeschlossen bezogen auf das Gesamtgewicht des Arzneimittels vorhanden ist.

14. Verwendung nach einem der vorangegangenen Ansprüche, **dadurch gekennzeichnet, dass** das Arzneimittel außerdem einen pharmazeutisch annehmbaren Träger, welcher für eine Verabreichung auf oralem, topischem äußerlichem, enteralem oder parenteralem Wege angepasst ist, umfasst.

15. Verwendung nach einem der vorangegangenen Ansprüche, **dadurch gekennzeichnet, dass** das Arzneimittel außerdem einen pharmazeutisch annehmbaren Träger, der für eine Verabreichung auf oralem Wege angepasst ist, umfasst.

16. Verwendung, außer einem therapeutischen Behandlungsverfahren, von wenigstens einem unverseifbaren Anteil eines pflanzlichen Öls, wie in Anspruch 4 definiert, für die Herstellung einer kosmetischen Zusammensetzung, die wenigstens einen aus kosmetischer Sicht annehmbaren Träger umfasst, zum Auftragen auf die Haut, die benachbarten Schleimhäute und/oder jegliche sichtbare epidermale (auf der Hautoberfläche befindliche) Strukturen oder Formationen, und welche für die kosmetischen Behandlung von Narben der Haut, der benachbarten Schleimhäute und/oder jeglicher sichtbarer epidermaler (auf der Hautoberfläche befindlicher) Strukturen oder Formationen bestimmt ist, mit der Maßgabe, dass die kosmetische Zusammensetzung keine Pollenextrakte umfasst.

17. Verwendung, außer einem therapeutischen Behandlungsverfahren, von wenigstens einem unverseifbaren Anteil eines pflanzlichen Öls, ausgewählt aus der Gruppe bestehend aus dem unverseifbaren Anteil von Avocadoöl, dem unverseifbaren Anteil von Sojaöl und den Mischungen von diesen Letzteren, für die Herstellung einer kosmetischen Zusammensetzung, die wenigstens einen aus kosmetischer Sicht annehmbaren Träger umfasst, zum Auftragen auf die Haut, die benachbarten Schleimhäute und/oder jegliche sichtbare epidermale (auf der Hautoberfläche befindliche) Strukturen oder Formationen, und welche für die Behandlung der intrinsischen Alterung der Haut, der benachbarten Schleimhäute und/oder jeglicher sichtbarer epidermaler (auf der Hautoberfläche befindlicher) Strukturen oder Formationen bestimmt ist, mit der Maßgabe, dass die kosmetische Zusammensetzung keinen Pollenextrakt umfasst.

18. Verwendung, außer einem therapeutischen Behandlungsverfahren, von wenigstens einem unverseifbaren Anteil eines pflanzlichen Öls, ausgewählt aus der Gruppe bestehend aus dem unverseifbaren Anteil von Avocadoöl, dem unverseifbaren Anteil von Sojaöl und den Mischungen von diesen Letzteren, für die Herstellung einer kosmetischen Zusammensetzung, die wenigstens einen aus kosmetischer Sicht annehmbaren Träger umfasst, zum Auftragen auf die Haut, die benachbarten Schleimhäute und/oder jegliche sichtbare epidermale (auf der Hautoberfläche befindliche) Strukturen oder Formationen, und welche für die kosmetische Behandlung der Haut, der benachbarten Schleimhäute und/oder jeglicher sichtbarer epidermaler (auf der Hautoberfläche befindlicher) Strukturen oder Formationen, die einer aktinischen Strahlung ausgesetzt worden sind, bestimmt ist, mit der Maßgabe, dass die kosmetische Zusammensetzung keinen Pollenextrakt umfasst.

19. Verwendung nach einem der Ansprüche 16 bis 18, **dadurch gekennzeichnet, dass** der unverseifbare Anteil eines pflanzlichen Öls der unverseifbare Anteil von Avocadoöl ist.

20. Verwendung nach einem der Ansprüche 16 bis 18, **dadurch gekennzeichnet, dass** der unverseifbare Anteil eines pflanzlichen Öls der unverseifbare Anteil von Öl von getrockneter Avocado ist.

21. Verwendung nach Anspruch 19 oder 20, **dadurch gekennzeichnet, dass** der unverseifbare Anteil von Avocado wenigstens dessen an furanischen Derivaten angereicherte Fraktion (Fraktion H), dessen an polyhydroxylierten Fettalkoholen angereicherte Fraktion (Fraktion I) oder eine Mischung von diesen Fraktionen umfasst.

22. Verwendung nach einem der Ansprüche 16 bis 18, **dadurch gekennzeichnet, dass** der unverseifbare Anteil eines pflanzlichen Öls der unverseifbare Anteil von Sojaöl ist.

23. Verwendung nach einem der Ansprüche 16 bis 18, **dadurch gekennzeichnet, dass** der unverseifbare Anteil eines pflanzlichen Öls eine Mischung des unverseifbaren Anteils von Avocadoöl und des unverseifbaren Anteils von Sojaöl ist, wobei das Gewichtsverhältnis des unverseifbaren Anteils von Avocadoöl und des unverseifbaren Anteils von Sojaöl zwischen 0,1 und ungefähr 9 eingeschlossen liegt.

24. Verwendung nach einem der Ansprüche 16 bis 18, **dadurch gekennzeichnet, dass** der unverseifbare Anteil eines pflanzlichen Öls an Phytosterolen, Tocopherolen, Tocotrienolen, terpenischen und triterpenischen Kohlenwasserstoffen und natürlichen Antioxidationsmitteln reiche Fraktionen enthält.

25. Verwendung, außer einem therapeutischen Behandlungsverfahren, von wenigstens einem unverseifbaren Anteil eines pflanzlichen Öls, wie in einem der Ansprüche 4 bis 11 definiert, für die Herstellung einer kosmetischen Zusammensetzung, die für die kosmetische, enthaarende Behandlung der Haut bestimmt ist, zum Auftragen auf die Haut, und welche wenigstens einen aus kosmetischer Sicht annehmbaren Träger umfasst.

26. Verwendung nach einem der Ansprüche 16 bis 25, **dadurch gekennzeichnet, dass** der unverseifbare Anteil eines pflanzlichen Öls in der Zusammensetzung entsprechend einem Anteil zwischen ungefähr 0,1 und ungefähr 10 Gew.-% eingeschlossen bezogen auf das Gesamtgewicht der kosmetischen Zusammensetzung vorhanden ist.

## Claims

1. Use of at least one unsaponifiable component of vegetable oil with a stimulating effect on the expression of TGF-β for the preparation of a medicament intended for the treatment of pahtologies selected from the group composed of lyme arthritis, psoriatic arthritis and osteoporosis.

2. Use according to Claim 1, **characterized in that** the medicament is intended to stimulate the expression of the isoforms TGF-β1 and TGF-β2, via the DNA sequences situated between -1132 and -732 bp of the promoter of the isoform TGF-β1.

3. Use according to Claim 1 or 2, **characterized in that** the said medicament is intended to also stimulate the expression of the plasminogen activator inhibitor PAI-1.

4. Use according to Claims 1 to 3, **characterized in that** the unsaponifiable component of vegetable oil is chosen from the group consisting of the unsaponifiable component of avocado oil, the unsaponifiable component of soya bean oil, the unsaponifiable component of lupin oil and mixtures of the latter.

5. Use according to Claim 4, **characterized in that** the unsaponifiable component of vegetable oil is the unsaponifiable component of avocado oil.

6. Use according to Claim 5, **characterized in that** the unsaponifiable component of vegetable oil is the unsaponifiable component of dry-avocado oil.

7. Use according to Claim 5 or 6, **characterized in that** the unsaponifiable component of avocado comprises at least its fraction enriched with furan derivatives (fraction H), its fraction enriched with polyhydroxylated fatty alcohols (fraction I) or a mixture of these fractions.

8. Use according to Claim 4, **characterized in that** the unsaponifiable component of vegetable oil is the unsaponifiable component of soya bean oil.

9. Use according to Claim 4, **characterized in that** the unsaponifiable component of vegetable oil is the unsaponifiable component of lupin oil.

10. Use according to Claim 4, **characterized in that** the unsaponifiable component of vegetable oil is a mixture of unsaponifiable component of avocado oil and of unsaponifiable component of soya bean oil, the weight ratio of unsaponifiable component of avocado oil to the unsaponifiable component of soya bean oil being between about 0.1 and about 9.

11. Use according to any one of Claims 1 to 3, **characterized in that** the unsaponifiable component of vegetable oil contains fractions rich in phytosterols, tocopherols, tocotrienols, terpenic and triterpenic hydrocarbons, and natural antioxidants.

12. Use according to Claim 11, **characterized in that** the unsaponifiable component of vegetable oil is chosen from the group consisting of the unsaponifiable component of canola, rapeseed, sunflower, palm, maize, sesame or wheatgerm oil, or the unsaponifiable component of soya bean oil, and mixtures of the latter.

13. Use according to any one of the preceding claims, **characterized in that** the unsaponifiable component of vegetable oil is present in the medicament in a proportion of between about 1 and about 80% by weight, relative to the total weight of the medicament.

14. Use according to any one of the preceding claims, **characterized in that** the medicament comprises, in addition, a pharmaceutically acceptable excipient suitable for administration by the oral, external topical, enteral or parenteral route.

15. Use according to any one of the preceding claims, **characterized in that** the medicament comprises, in addition, a pharmaceutically acceptable excipient suitable for administration by the oral route.

16. Use, other than a method of therapeutic treatment, of at least one unsaponifiable component of vegetable oil as defined in Claim 4 for the preparation of a cosmetic composition comprising at least one cosmetically acceptable vehicle, to be applied to the skin, the neighbouring mucous membranes and/or the superficial body growths, and intended for the cosmetic treatment of scars on the skin, of the neighbouring mucous membranes and/or of the superficial body growths, under the proviso that the cosmetic composition does not comprise pollen extract.

17. Use, other than a method of therapeutic treatment, of at least one unsaponifiable component of vegetable oil chosen from the group consisting of the unsaponifiable component of avocado oil, the unsaponifiable component of soya bean oil and mixtures of the latter for the preparation of a cosmetic composition comprising at least one cosmetically acceptable vehicle, to be applied to the skin, the neighbouring mucous membranes and/or the superficial body growths, and intended for the treatment of intrinsic ageing of the skin, of the neighbouring mucous membranes and/or of the superficial body growths, under the proviso that the cosmetic composition does not comprise pollen extract.

18. Use, other than a method of therapeutic treatment, of at least one unsaponifiable component of vegetable oil chosen from the group consisting of the unsaponifiable component of avocado oil, the unsaponifiable component of soya bean oil and mixtures of the latter for the preparation of a cosmetic composition comprising at least one cosmetically acceptable vehicle, to be applied to the skin, the neighbouring mucous membranes and/or the superficial body growths, and intended for the cosmetic treatment of the skin, of the neigbouring mucous membranes and/or of the superficial body growths which have been subjected to actinic radiation, under the proviso that the cosmetic composition does not comprise pollen extract.

19. Use according to any one of Claims 16 to 18, **characterized in that** the unsaponifiable component of vegetable oil is the unsaponifiable component of avocado oil.

20. Use according to any one of Claims 16 to 18, **characterized in that** the unsaponifiable component of vegetable oil is the unsaponifiable component of dry-avocado oil.

21. Use according to Claim 19 or 20, **characterized in that** the unsaponifiable component of avocado comprises at least its fraction enriched with furan derivatives (Fraction H), its fraction enriched with polyhydroxylated fatty alcohols (Fraction I) or a mixture of these fractions.

22. Use according to any one of Claims 16 to 18, **characterized in that** the unsaponifiable component of vegetable oil is the unsaponifiable component of soya bean oil.

23. Use according to any one of Claims 16 to 18, **characterized in that** the unsaponifiable component of vegetable oil is a mixture of unsaponifiable component of avocado oil and of unsaponifiable component of soya bean oil, the weight ratio of unsaponifiable component of avocado oil to the unsaponifiable component of soya bean oil being between 0.1 and about 9.

24. Use according to any one of Claims 16 to 18, **characterized in that** the unsaponifiable component of vegetable oil contains fractions rich in phytosterols, tocopherols, tocotrienols, terpenic and triterpenic hydrocarbons, and natural antioxidants.

25. Use, other than a method of therapeutic treatment, of at least one unsaponifiable component of vegetable oil as defined in any one of Claims 4 to 11 for the preparation of a cosmetic composition intended for the cosmetic depilatory treatment of the skin, to be applied to the skin, and comprising at least one cosmetically acceptable vehicle.

26. Use according to any one of Claims 16 to 25, **characterized in that** the unsaponifiable component of vegetable oil is present in the composition in a proportion of between about 0.1 and about 10% by weight, relative to the total weight of the cosmetic composition.
